# FASCICULE DE BREVET EUROPEEN

(11) **EP 1 372 618 B1**
(45) Date de publication et mention de la délivrance du brevet: **05.12.2012**
(21) Numéro de dépôt: 02724379.9
(22) Date de dépôt: 26.03.2002
(51) Int. Cl.: A61K 9/51

(54) **SUSPENSION COLLOIDALE DE NANOPARTICULES A BASE D' UN COPOLYMERE AMPHIPHILE**
KOLLOIDALE SUSPENSION VON NANOPARTIKELN AUF BASIS EINES AMPHIPHILEN COPOLYMERES
COLLOIDAL SUSPENSION OF NANOPARTICLES BASED ON AN AMPHIPHILIC COPOLYMER

(30) Priorité: 02.04.2001 FR 0104512
(43) Date de publication de la demande: 02.01.2004
(73) Titulaire: FLAMEL TECHNOLOGIES, 69693 Venissieux Cédex (FR)
(72) Inventeur: SOULA, Gérard, F-69330 MEYZIEU (FR); BRYSON, Nathan, F-69390 MILLERY (FR)
(74) Mandataire: Cabinet Plasseraud
(86) Numéro de dépôt international: PCT/FR2002/001045
(87) Numéro de publication internationale: WO 2002/078677

(56) Documents cités:
- FR-A- 2 786 098
- GB-A- 2 240 547
- M. YOKOHAMA ET AL.: "Incorporation of water-insoluble anticancer drug into polymeric micelles and control of their particle size" JOURNAL OF CONTROLLED RELEASE, vol. 55, no. 2-3, 13 novembre 1998 (1998-11-13), pages 219-229, XP004143517 Amsterdam (NL)

## Description

Le domaine de la présente invention est celui des Nanoparticules de Vectorisation (PV), utiles pour l'administration de principes actifs (PA). Ces derniers sont, de préférence, des médicaments ou des nutriments pour l'administration à un organisme animal ou humain par voie orale ou nasale, vaginale, oculaire, sous-cutanée, intraveineuse, intramusculaire, intradermique, intrapéritonéale, intracérébrale, etc. En termes de nature chimique, les PA plus particulièrement concernés par l'invention sont hydrophiles, par exemple des protéines, des glycoprotéines, des peptides, des polysaccharides, des lipopolysaccharides, ou des polynucléotides.

La présente invention concerne, plus précisément, des suspensions colloïdales de Nanoparticules de Vectorisation (**PV**), à base de blocs de polyaminoacides et polymères hydrophiles du type PolyAlkylène-Glycol (PAG), de préférence PolyEthylène-Glycol (PEG).

La présente invention vise aussi bien des **PV** en tant que telles que des systèmes vecteurs de **PA,** constitués par des **PV** chargées en **PA.**

La présente invention a également trait à des solides pulvérulents comprenant ces **PV.**

L'invention concerne, également, des procédés de préparation desdites suspensions colloïdales de particules, chargées en **PA.**

L'association de **PA** avec des **PV** a notamment pour but de modifier leur durée d'action et/ou de les acheminer au lieu du traitement et/ou augmenter la biodisponibilité desdits **PA.** De nombreuses techniques d'association ont déjà été proposées. De telles techniques visent, d'une part, à permettre le transport du **PA** jusqu'à son site d'action thérapeutique, tout en le protégeant contre les agressions de l'organisme (hydrolyse, digestion enzymatique, etc.) et, d'autre part, à contrôler la libération du **PA** sur son site d'action, afin de maintenir la quantité disponible pour l'organisme au niveau désiré. Les **PA** concernés par ces avatars de transport et de séjour dans l'organisme sont, par exemple, des protéines mais peuvent être également des produits tout autre, des molécules organiques d'origine synthétique ou naturelle. La revue de M.J. HUMPHREY (Delivery system for peptide Drugs, éditée par S. DAVIS et L. ILLUM, Plenum Press, N.Y. 1986) fait état de la problématique concernant l'amélioration de la biodisponibilité des **PA** et l'intérêt des systèmes de vectorisation et de libération contrôlée.

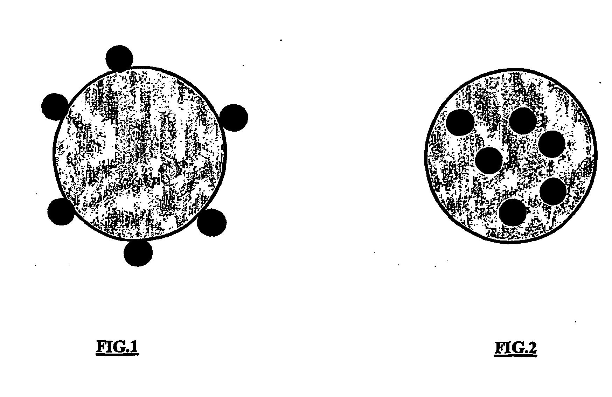

En fait, on distingue deux types principaux de systèmes de vectorisation et de libération contrôlée de **PA,** qui sont caractérisés par le mode d'association du **PA** avec les **PV,** à savoir :
- l'association par adsorption, illustrée par la figure 1 annexée,
- et l'association par encapsulation (ou par enrobage), illustrée par la figure 2 annexée.

L'association du **PA** aux **PV** par adsorption spontanée, est celle qui est concernée par la présente invention. Généralement, les techniques d'adsorption spontanée sont moins agressives vis à vis des **PA** que les techniques d'association par encapsulation, dans lesquelles on a souvent recours à des solvants et/ou des tensioactifs, ainsi que des étapes de procédé (émulsification, évaporation, distillation), propres à dénaturer les **PA,** et en particulier les **PA** de nature protéique (préservation de la structure secondaire native), qui constituent la majorité des **PA** usuels visées. Dans le cas d'une association par adsorption, la libération s'opère par désorption.

Au-delà des modes d'association/relargage du **PA** vis à vis des **PV,** les matériaux constitutifs des **PV** doivent présenter des propriétés d'usage particulières. Finalement, le cahier des charges que l'on souhaite obtenir pour les **PV** est particulièrement exigeant et comprend, notamment, les spécifications suivantes.
1 La première spécification recherchée pour les **PV** est :
   o d'une part, que les **PV** s'associent aisément avec les **PA** pour former des systèmes **PV-PA** qui permettent une libération et une action prolongées du **PA** in vivo (par exemple, une durée d'action d'au moins 24 heures pour un **PA** = insuline),
   o et d'autre part, que ces **PV** (chargées ou non en **PA**) forment des suspensions aqueuses stables (par exemple au moins pendant plusieurs mois), sans l'aide de solvant organique et/ou de tensioactif; c'est-à-dire les **PV** se maintiennent en suspension et ne floculent pas.
2 Les **PV** doivent être constituées d'un (co)polymére biocompatible, éliminable (par excrétion) et/ou biodégradable rapidement en produits non toxiques pour l'organisme.
3 Il est également recherché que les **PV** aient une taille suffisamment faible pour pouvoir subir, en suspension dans un liquide, une filtration stérilisante par un filtre dont le diamètre des pores est inférieur ou égal à 0, 2 µm.
4 Il est souhaitable que les **PV** et les systèmes **PV-PA** puissent être obtenus par un procédé non dénaturant pour le **PA.**
5 Les **PV** doivent, avantageusement, permettre de contrôler la vitesse de libération du **PA.**
6 Une autre spécification importante est que les systèmes **PV-PA** puissent constituer d'excellents médicaments injectables. Cette aptitude améliorée de l'administration par injection -e.g. intraveineuse ou intramusculaire- ou « injectabilité » se caractérise par :
   (i) un volume injecté réduit (pour une dose thérapeutique donnée),
   (ii) une viscosité faible.

   Ces deux propriétés sont satisfaites lorsque la dose thérapeutique de **PA** est associée à une quantité minimale de **PV.** En d'autres termes, les **PV** doivent avoir un fort taux de chargement en **PA.**
7 Le coût propre aux **PV** dans une préparation injectable doit être réduit et là encore il convient que les PV aient un fort taux de chargement en **PA.** En définitive, la faible taille et un fort taux de chargement sont des spécifications majeures recherchées pour les **PV.**
8 Il est également avantageux que le polymère, constitutif des **PV,** n'induise pas de réponse immunitaire.
9 Enfin, il est avantageux que les **PV** aient une durée de vie dans l'organisme au moins égale au temps de libération recherchée pour le **PA.**

Les propositions techniques antérieures (a) à (g), décrites infra, ont tenté de satisfaire, en vain, à l'ensemble de ces spécifications.
(a) Le brevet US-A-5 286 495 concerne un procédé d'encapsulation par vaporisation de protéines en phase aqueuse, à l'aide de matériaux ayant des charges opposées, à savoir : l'alginate (chargé négativement) et la polylysine (chargée positivement). Ce procédé de fabrication permet de produire des particules de taille supérieure à 35 µm.
(b) Par ailleurs, les techniques d'émulsion sont couramment utilisées pour préparer des microparticules chargées de **PA.** Par exemple, les demandes de brevets WO 91/06286, WO 91/06287 et WO 89/08449 divulguent de telles techniques d'émulsion dans lesquelles on a recours à des solvants organiques pour solubiliser des polymères, par exemple de type polylactique. Mais il s'est avéré que les solvants peuvent être dénaturants, notamment pour les **PA** peptidiques ou polypeptidiques.
(c) On connaît, également, des **PV** biocompatibles appelées protéinoïdes, décrites dès 1970 par X. FOX et K. DOSE dans « Molecular Evolution and the origin of Life », Ed. Marcel DEKKER Inc (1977). Ainsi, la demande de brevet WO 88/01213 propose un système à base d'un mélange de polypeptides synthétiques dont la solubilité dépend du pH. Pour obtenir les microparticules matricielles selon cette invention, ils solubilisent le mélange de polypeptides, puis avec un changement de pH, ils provoquent la précipitation de particules protéinoides. Lorsque la précipitation s'effectue en présence d'un **PA,** celui-ci est encapsulé dans la particule.
(d) On mentionnera également, pour mémoire, le brevet US 4 351 337 qui relève d'un domaine différent de celui de la vectorisation de **PA** propre à l'invention. Ce brevet divulgue des implants massiques fixés et localisés à des endroits bien précis de l'organisme. Ces implants sont des tubes ou des capsules creuses de taille microscopique (160 µm et de longueur égale à 2 000 µm), constitués de copolymères de copoly(aminoacides) -e.g. poly(acide glutamique-leucine) ou poly(benzylglutamate-leucine)-obtenus par copolymérisation de monomères de N-carboxyanhydrides d'aminoacides (NCA). L'inclusion d'un **PA** s'opère par une technique d'évaporation de solvant d'un mélange de polymère et de **PA.** Le brevet US 4 450 150 appartient à la même famille que le brevet US 4 351 337 étudié ci-dessus et a essentiellement le même objet. Les **PAA** constitutifs sont des poly(acide glutamique-éthylglutamate).
(e) La demande de brevet PCT/FR WO 97/02810 divulgue une composition pour la libération contrôlée de principes actifs, comprenant une pluralité de particules lamellaires d'un polymère biodégradable, au moins en partie cristallin (polymère d'acide lactique) et d'un **PA** adsorbé sur lesdites particules. Dans ce cas, la libération du principe actif s'opère par désorption.
(f) La demande PCT WO 96/29991 a pour objet des particules de polyaminoacides utiles pour la vectorisation de **PA** tels que l'insuline. Ces particules ont une taille comprise entre 10 et 500 nm.
   Les particules selon le WO 96/29991 se forment spontanément par mise en contact de PAA avec une solution aqueuse. Les PAA comprennent un bloc hydrophobe formé par des monomères aminoacides neutres et hydrophobes AAO (polyLeu) et un bloc hydrophile formé par des monomères ionisables et hydrophiles AAI (polyGlu).
(g) L'EP 0 583 955 divulgue des micelles polymère capables de piéger physiquement des PA hydrophobes. Ces micelles sont constituées par des copolymères bloc comportant un bloc hydrophile constitué par du PolyEthylèneGlycol (PEG) et un bloc hydrophobe constitué par un PolyAminoAcide, par exemple : PEG/polyAANO (AANO = Amino-Acide Neutre hydrophobe).
   L'AANO peut être : Leu, Val, Phe, Bz-O-Glu, Bz-O-Asp, ce dernier étant préféré. Les principes actifs **PA** hydrophobes piégés dans ces micelles PEG/polyAANO sont e.g.: adriamycine, indométhacine, daunomycine, methotrexate, mitomycine.
   Dans cette demande de brevet, seuls sont exemplifiées les micelles à base de PEG/polyGlu-O-Bz. Or, il est à noter que ces esters Glu-O-Bz ne sont pas stables à l'hydrolyse en milieu aqueux. En outre, l'hydrolyse enzymatique de ces produits forme des dérivés non naturels du benzène potentiellement toxiques. Par ailleurs, il n'est nullement question dans ce document de particules constituées par un copolymère bloc PEG/polyAANO, dont le coeur est formé par le polyamino acide neutre hydrophobe et comprenant une chevelure externe hydrophile à base de PEG, ces particules étant aptes à s'associer avec des PA hydrophiles et à les libérer in vivo.
(h) Le FR-A-2786098 divulgue des nanoparticules et des microparticules de vectorisation de principes actifs. Ces nanoparticules et ces microparticules sont à base de polyaminoacide de type polyleucine-polyglutamate. Il s'agit donc exclusivement de copolyaminoacides linéaires amphiphiles dibloc.
(i) L'article « Incorporation of water-soluble anticancer drug into polymeric micelles and control of their particle size » de M. Yokohama et al. concerne l'incorporation d'un principe actif anti-cancéreux le KRN 5500 dans des micelles polymères formées par des copolymères bloc PEG/poly(beta-benzyle-L-aspartate) dénommé en abrégé PEG-PBLA, PEG/poly (Asp)-poly(beta-benzyle-L-aspartate) également dénommé PEG-PBLA-PAsp, PEG-(poly(beta-cétyl-L-aspartate-(-poly(beta-benzyle-L-aspartate) également dénommé PEG-PC₁₆LA-PBLA. Les micelles de PEG-polyaminoacide divulgués dans cet article ne comportent pas de polyaminoacide amphiphile. Si le polyaminoacide du copolymère PEG-polyaminoacide est un polyaminoacide amphiphile, ce copolymère ne produit pas de micelle. L'incorporation du principe actif dans les micelles telle que décrite dans ce document, s'effectue à l'état dissous dans un solvant organique de type DMSO ou DMF.

On sait par ailleurs que les PEG ne sont pas biodégradables et sont même susceptibles de protéger des **PV** d'une dégradation enzymatique, nuisant ainsi à la biodégradabilité in vivo des **PV**, l'une des caractéristiques essentielles recherchées dans le cadre de la présente invention.

Il ressort donc de ce qui précède que les propositions techniques antérieures susdécrites satisfont incomplètement aux spécifications du cahier des charges indiqué supra, et, en particulier les spécifications 1 (durée de libération et d'action in vivo prolongées et stabilité en suspension aqueuse des PV), 2 (biodégradabilité), 4 (obtention non dénaturante), 5 (contrôle vitesse libération), 8 (absence de réponse immunitaire).

Dans cet état de fait, l'un des objectifs essentiels est de pouvoir fournir de nouvelles **PV** (du type adsorption/désorption) qui forment spontanément, et sans l'aide de tensioactifs ni de solvants organiques, des suspensions aqueuses stables de **PV** et adaptées à la vectorisation de **PA** (notamment des protéines telles que l'insuline), et surtout qui permettent d'augmenter significativement la durée de libération et d'action in vivo du **PA,** par rapport aux systèmes de vectorisation connus dans l'art antérieur et présentés ci-dessus (a-g, vide supra).

Un autre objectif essentiel de la présente invention est de fournir de nouvelles **PV** en suspension aqueuse colloïdale stable (notamment à l'hydrolyse) ou sous forme pulvérulente et à base de copolymères bloc PolyAlkylèneGlycol (PAG)/poly(aminoacides) (PAA), ces nouvelles **PV** se devant de satisfaire au mieux aux spécifications 1 à 9 du cahier des charges susvisé.

Un autre objectif essentiel de l'invention est de perfectionner les particules divulguées dans la demande EP 0 583 955.

Un autre objectif essentiel de l'invention est de fournir une suspension de **PV,** qui, bien que possédant une couronne extérieure de PEG, soit cependant facilement biodégradable.

Un autre objectif essentiel de l'invention est de fournir une suspension nouvelle de **PV** dont on maîtrise parfaitement les caractéristiques, notamment en termes du taux de chargement en **PA** et en termes de contrôle de cinétique de libération du **PA.**

Un autre objectif essentiel de l'invention est de fournir des suspensions de **PV** médicamenteuses stables et administrables à l'homme ou l'animal, par voie orale ou parentérale, par exemple.

Un autre objectif essentiel de l'invention est de fournir une suspension colloïdale aqueuse ou un solide pulvérulent comprenant des particules de vectorisation de principes actifs satisfaisant aux spécifications visées ci-dessus et qui constitue une forme galénique appropriée et convenable pour une administration, par exemple orale, à l'homme ou l'animal.

Un autre objectif essentiel de l'invention est de proposer un procédé de préparation de particules (sèches ou en suspension dans un liquide) de **PAA** utiles, notamment, comme vecteurs de principes actifs (notamment protéines telles que l'insuline), ledit procédé se devant d'être plus simple à mettre en oeuvre, non dénaturant pour les principes actifs et devant en outre toujours permettre une maîtrise fine de la granulométrie moyenne des particules obtenues.

Un autre objectif essentiel de l'invention est l'utilisation des susdites particules en suspension aqueuse ou sous forme solide pour la préparation de médicaments (e.g. vaccins), en particulier pour administration notamment orale, nasale, vaginale, oculaire, sous-cutanée, intraveineuse, intra-musculaire, intradermique, intra-péritonéale, ou intra-cérébrale, les principes actifs hydrophiles de ces médicaments pouvant être, notamment, des protéines, des glycoprotéines, des peptides, des polysaccharides, des lipopolysaccharides, des oligonucléotides et des polynucléotides.

Un autre objectif de la présente invention est de fournir un médicament, du type système à libération prolongée de principes actifs, qui soit aisé et économique à produire et qui soit, en outre, biocompatible et apte à assurer un très haut niveau de biodisponibilité du **PA**.

Un autre objectif essentiel de l'invention est de fournir un système de vectorisation de vaccin, qui soit non immunogène intrinsèquement et en combinaison avec un ou plusieurs antigènes.

Ces objectifs (parmi d'autres) sont atteints par la présente invention qui concerne, tout d'abord, une suspension colloïdale de particules submicroniques susceptibles d'être utilisées, notamment pour la vectorisation de principe(s) actif(s) (**PA**), ces particules étant des arrangements supramdléculaires individualisés. Cette suspension a ceci de particulier, que lesdites particules :
- sont à base d'au moins un copolymère amphiphile comprenant :
   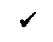 au moins un bloc de polymère(s) hydrophile(s) du type polyéthylène-glycol (PEG) ;
   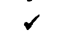 et au moins un copolyaminoacide(s) (PAA) amphiphile linéaire(s), à enchaînements α-peptidiques; ce copolymère étant constitué de chaînes de copolyméres "triblocs linéaires" PEG/AminoAcide hydrophIle (AAI)/ AminoAcide hydrophObe(AAO),
- et sont aptes à s'associer en suspension colloïdale à l'état non dissous, avec au moins un **PA** et à libérer celui-ci, notamment in vivo, de manière prolongée et/ou retardée.

L'un des fondements inventifs de ces nouvelles particules de vectorisation **PV,** en suspension aqueuse colloïdale stable ou à l'état de solide pulvérulent, tient à la sélection originale d'un copolymère PAG/PAA amphiphile, par exemple un terpolymère bloc
[polymère hydrophile/ polyaminoacide hydrophile /polyaminoacide hydrophobe],
permettant d'obtenir des particules de taille nanométrique (10-500nm), qui forment une suspension colloïdale aqueuse stable, tant vis à vis de l'hydrolyse que de la floculation, en l'absence de tensioactifs et/ou de solvants et qui puissent se lier à des **PA** par adsorption et libérer ces **PA** par désorption. Dans le cas de la présente invention, l'adsorption se fait naturellement et spontanément lorsque les particules colloïdales et le **PA** sont mis en contact dans un milieu aqueux. L'adsorption dépend de la nature du support (PV) et la quantité de support accessible au **PA.**

L'un des intérêts majeurs de la présente invention est qu'elle conduit à un système **PV-PA** ayant une durée d'action in vivo augmentée de manière significative (par exemple de 10h lorsque **PA =** insuline), par rapport aux systèmes connus, et notamment par rapport au système décrit dans la demande de brevet WO 96/29991 et comprenant des **PV** poly(Glu)/polyLeu.

Par ailleurs, le fait que les **PV** soient en partie constituées d'un polyaminoacide hydrophile offre l'avantage d'une possibilité aisée de dégradation des **PV** par hydrolyse enzymatique, ce qui facilite leur élimination de l'organisme.

De préférence, les Aminoacides du (ou des) copolyaminoacide(s) (PAA) amphiphile (s) composant les particules sont d'au moins deux types :
➢ un premier type comprenant au moins un AminoAcide hydrophile(AAI) ;
➢ un deuxième type comprenant au moins un AminoAcide hydrophObe(AAO).

En pratique, le copolyaminoacide(s) (PAA) amphiphile (s) composant les particules comporte avantageusement au moins une séquence globalement hydrophile et au moins une séquence globalement hydrophobe.

Conformément à l'invention, la structure des copolymères amphiphiles et la nature des acides aminés AAI et AAO, sont choisies de telle façon que :
- les chaînes de polymères se structurent spontanément sous forme de particules **(PV)** de petite taille,
- les particules forment une suspension colloïdale stable dans l'eau et en milieu physiologique,
- les PV s'associent avec des protéines ou autres **PA** en milieu aqueux (en l'absence de solvant organique et/ou de tensioactif), par un mécanisme spontané et non dénaturant pour le **PA,**
- les **PV** libèrent les **PA** du complexe d'association **PA-PV** dans les conditions physiologiques, et plus précisément in vivo, avec des profils pharmacocinétique et pharmacodynamique idoines pour des utilisations à titre de médicaments; la cinétique de libération est fonction de la nature du copolymère PAG/PAA (=PolyAAI/PolyAAO )précurseur des **PV.**

Ainsi, en jouant sur la structure bloc particulière du copolymère, on peut contrôler les phénomènes d'association et de libération du **PA** sur le plan cinétique et quantitatif.

La fraction hydrophobe polyAAO participe à l'agrégation des chaînes polymères, ce qui est au coeur de la formation des **PV.**

Suivant une modalité remarquable de l'invention:
- l'(ou les)aminoacide(s) hydrophlle(s) (AAI), est sont sélectionné(s) dans le groupe comprenant :
   ➢ les AminoAcides à chaine(s) ionisable(s), au moins partiellement ionisés, de préférence Glu et/ou Asp et leurs sels et/ou Lys; naturels et non-naturels
   ➢ et leurs mélanges;
- et l'(ou les) AminoAcide(s) hydrophObe(s) (AAO), est sont sélectionné(s) dans le groupe comprenant :
   ➢ les acides aminés neutres naturels, avantageusement ceux du sous-groupe : Leu, Ile, Val, Ala, Pro, Phe, et leurs mélanges ;
   ➢ des acides aminés neutres, rares ou synthétiques, avantageusement ceux du sous-groupe : norleucine, norvaline et leurs mélanges;
   ➢ des dérivés des , acides aminés polaires avantageusement ceux du sous-groupe : glutamate de méthyle, glutamate d'éthyle, aspartate de benzyle, N-acétyllysine et leurs mélanges;
   ➢ et leurs mélanges.

Selon l'invention, le(s) copolyaminoacide(s) (PAA) amphiphile(s) composant le copolymère amphiphile constitutif des particules, présente(nt) une structure "bloc".

Ce copolyaminoacide(s) (PAA) amphiphile(s) comporte au moins une séquence globalement hydrophile et au moins une séquence globalement hydrophobe, qui lui confèrent son amphiphilie.

Le PAA amphiphile "bloc" préféré comporte avantageusement:
o au moins une séquence globalement hydrophile essentiellement constituée d'aminoacides AAI, et ayant une longueur absolue supérieure ou égale à 5 monomères AAI, de préférence supérieure ou égale à 200 monomères AAI, et plus préférentiellement encore comprise entre 10 et 50 monomères AAI
o et au moins une séquence globalement hydrophobe essentiellement constituée d'aminoacides AAO, ayant une longueur absolue supérieure ou égale à 5 monomères AAO, de préférence supérieure ou égale à 10 monomères AAO, et plus préférentiellement comprise entre 10 et 50 monomères AAO.

S'agissant du PAG hydrophile - de préférence le PEG -, il se présente avantageusement sous la forme d'un bloc dont la longueur absolue est supérieure ou égale à 5 monomères, de préférence comprise entre 5 et 120 monomères, et plus préférentiellement encore comprise entre 5 et 50 monomères. Il est à noter que les blocs de PAG (e.g. PEG) peuvent être des homopolymères ou des copolymères, les homopolymères étant plus particulièrement appréciés.

De manière plus préférée encore, il est du mérite de la demanderesse d'avoir choisi, à titre de matériau constitutif des **PV,** une classe particulière de terpolymères bloc : *polymère bydropbile*/*polyaminoacide hydrophile*/*polyaminoacide hydrophobe,* qui sont amphiphiles et chargés. Cette amphiphilie permet d'obtenir des propriétés nouvelles et surprenantes et notamment celles évoquées ci-dessus. Ainsi, les **PV** selon l'invention forment des suspensions aqueuses stables, en l'absence de tout tensioactif et de tout solvant organique et à des pH physiologiques. De plus, grâce au choix de la structure en tribloc, incorporant une séquence hydrophile à base d'acides aminés entre la partie PAG et la partie hydrophobe, les **PV** sont facilement dégradables in vivo par une réaction d'hydrolyse enzymatique. C'est l'un des points clés du système terpolymère selon le mode préféré de réalisation de l'invention.

En outre, les associations **PV-PA** se font spontanément et surtout permettent une libération et donc une action in vivo pendant de très longues durées (par exemple, 30 heures et plus avec une protéine telle que l'insuline). Ainsi, le temps d'action du **PA** in vivo est suffisamment long pour augmenter significativement la couverture thérapeutique et par suite améliorer le confort du patient.

Suivant un exemple de réalisation concret de l'invention, les particules sont constituées de chaînes de copolymères amphiphiles "triblocs linéaires" PEG/AAI/AAO, correspondant de préférence à la formule suivante : 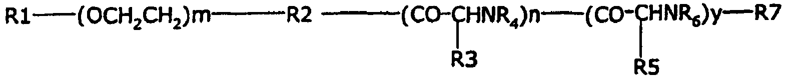 dans laquelle :
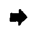 R1 = H, alkyle linéaire ou branché en C₁-C₂₀ (substitué ou non), aryle de préférence benzyle (substitué ou non);
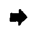 R2 = NH, CO-NH, CS-NH, R8-(CH₂)ₜ-R9 avec R8, R9 choisi indépendamment parmi = OCO, OCONH, NHCO, NHCONH, CONH, COO, NH, CO; t = 1-6, [NHCH(R1)CO-]ₓ;
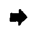 R3 = radicaux identiques ou différents le long de la chaîne et choisis parmi les groupements définissant des aminoacides hydrophiles, ionisables (naturels ou dérivés synthétiques) à savoir de préférence les groupements (CH2)ₚC₆H₄OM, (CH₂)ₚCO₂M, (CH₂)ₚN(H_{c}R1_{d})₃X avec p ≥ 1, de préférence = 1 ou 2; a et b sont des valeurs comprise entre 0 et 3 et a+b = 3 ; X étant de préférence un anion chlorure, bromure, sulfate, nitrate, hydrogeno-phosphate, acétate, lactate ;
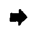 R4 = radicaux identiques ou différents le long de la chaîne et choisis parmi les groupements H, Me;
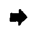 R5 = radicaux identiques ou différents le long de la chaîne et choisis parmi les groupements définissant des aminoacides hydrophobes (naturels ou dérivés synthétiques)à savoir de préférence les groupement s H, R1, (CH₂)_{q}C₆H₅, (CH₂)_{q} C₆H₄OR1, (CH₂)_{q} OR1, (CH₂)_{q} CO₂R1, (CH₂)_{q}CON(R1)₂ , avec q ≥ 1, de préférence = 1 ou 2;
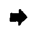 R6 = R4 ;
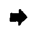 R7 = H, R1CO avec R1 tel que défini supra, alkyle linéaire ou branché en C₁-C₂₀ (substitué ou non), aryle de préférence benzyle (substitué ou non), hydroxyalkyle en C₁-C₆ , H, -(CH₂)_{w}OH, (CH₂)_{w}CO₂M, - (CH₂)_{w}(CHR1)₂OH, - (CH₂)_{w}NH₂, -(CH₂)_{y}C₆H₄OH, (CH₂)yCO-N(R1)₂; R10 = H, Me, (CH₂)ᵥOH 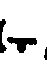 , avec w, z et v ≥ 1 et M = métal ou cation, typiquement un métal alcalin comme Na, Li, K, ou NH4, R1ₐNH_{b} ;
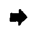 m > 1; n > 3.; y ≥ à 0; a+b = 4.

Conformément à l'invention, le fait d'avoir une séquence hydrophile polyaminoacide (polyAAI) dans le copolymère constitutif des **PV**, est une disposition particulièrement avantageuse en ce que cela améliore la biodégradabilité des **PV.** Cette séquence PolyAAI est préférablement disposée entre un bloc polymère hydrophile et une séquence hydrophobe polyaminoacide (polyAAO).

Les particules **PV** selon l'invention ont une taille moyenne comprise entre 10 et 500 nm, de préférence entre 10 et 200 nm. Au sens de l'invention, on entend, par taille ou granulométrie moyenne, le diamètre hydrodynamique moyen.

L'un des atouts de l'invention est d'être parvenu à un très bon contrôle de la granulométrie moyenne des ces entités et de leur répartition granulométrique. Le contrôle de l'autoassociation des chaînes de polymère et donc de la taille des PV, s'opère par l'intermédiaire de la composition en polyaminoacides, mais aussi, pour une même composition, par le choix d'une structure bloc et le procédé d'obtention. De cette manière, la taille des particules est extrêmement faible, de l'ordre de quelques nanomètres à quelques dizaines de nanomètres.

La suspension selon l'invention est aqueuse et stable.

La présente invention vise, non seulement des suspensions de particules nues, telles que définies ci-dessus, mais également des suspensions de particules comprenant au moins un principe actif **PA.**

Ces particules, associées ou non avec un **PA,** sont, avantageusement, sous forme dispersée dans un liquide aqueux, mais peuvent également être à l'état de solide pulvérulent, obtenu par solidification et mise sous forme de poudre de la suspension de **PV** telle que définie ci-dessus.

L'invention concerne donc, outre une suspension colloïdale aqueuse de **PV,** un solide pulvérulent comprenant des **PV et** obtenu à partir de la suspension selon l'invention.

Il est à noter que la nature de la distribution des groupements hydrophobes sur les chaînes de polymères est contrôlable de par la voie de synthèse choisie. A cet égard, il existe de nombreux schémas réactionnels conduisant aux polymères sélectionnés comme matière première pour l'obtention des **PV** selon l'invention.

Conformément à l'invention, on retient un mode particulier de préparation des **PV** et de la suspension de **PV.**

Ainsi, un autre objet essentiel de l'invention se rapporte à la préparation des particules sélectionnées (telles que décrites ci-avant), aussi bien sous forme de suspension colloïdale que sous forme de solide pulvérulent. Le procédé de préparation considéré consiste, essentiellement :
* à synthétiser des copolymères PAG/polyAAI/polyAAO précurseurs des **PV** et à les transformer en particules **PV** structurées ;
* éventuellement à purifier les particules ainsi produites ;
* éventuellement à isoler ces particules, de préférence par concentration, lyophilisation, filtration ou séchage.

Plus précisément, il s'agit, tout d'abord, d'un procédé de préparation du solide pulvérulent susvisé et formé par de particules structurées nanométriques susceptibles d'être utilisées, notamment pour la vectorisation de principe(s) actif(s), ces particules étant des arrangements supramoléculaires individualisés :
- à base d'au moins un copolymère amphiphile comprenant :
   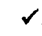 au moins une séquence hydrophobe de polyaminoacide(s) (PAA) linéaire(s), à enchaînements α-peptidiques, les aminoacides hydrophobes AAO constitutifs de ce bloc PAA étant identiques ou différents entre eux ;
   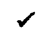 au moins une séquence hydrophile de polyaminoacide(s) (PAA) linéaire(s), à enchaînements α-peptidiques, les aminoacides hydrophobes AAI constitutifs de ce bloc PAA étant identiques ou différents entre eux ;
   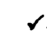 et au moins un bloc de polymère(s) hydrophile(s) du type polyalkylèneglycol (PAG), de préférence polyéthylène-glycol (PEG) ;
- aptes à s'associer en suspension colloïdale à l'état non dissous, avec au moins un PA et à libérer celui-ci, notamment in vivo, de manière prolongée et/ou retardée.

Ce procédé est caractérisé en ce que :
1) on fait réagir au moins un bloc PAG comprenant au moins un monomère alkylène-glycol, avec au moins une séquence hydrophile de PAA comprenant au moins un monomère aminoacide AAI hydrophile et avec au moins une séquence hydrophobe de PAA comprenant au moins un monomère aminoacide AAO hydrophobe, ce bloc de PAG et ces séquences comprenant chacun au moins une fonction réactive de façon à obtenir un copolymère amphiphile "bloc" PAG/polyAAI/polyAAO ;
2) on transfère le copolymère amphiphile bloc PAG/polyAAI/polyAAO obtenu à l'étape 1, dans un milieu non-solvant de la (des) fraction(s) hydrophobe(s) du copolymère amphiphile - de préférence dans l'eau -, ce qui conduit à la formation spontanée de particules de vectorisation (**PV**) ;
3) éventuellement on dialyse le milieu réactionnel pour purifier la suspension aqueuse de particules structurées ;
4) éventuellement on concentre cette suspension de l'étape 3 ;
5) éventuellement on associe au moins un principe actif **PA** avec les particules de l'étape 2, 3 ou 4 ;
6) on élimine le milieu liquide pour recueillir le solide pulvérulent comprenant les particules chargées ou non.

Au sens de l'invention, l'expression "non-solvant" signifie que les fractions du copolymère amphiphile considéré, ont par exemple une solubilité inférieure à 1g/l, à 25°C dans le milieu non-solvant considéré.

En fin d'étape 2, le milieu liquide ne forme pas une solution homogène, mais se sépare en une phase dispersée (PV) et une phase pauvre en copolymère amphiphile "bloc".

Les fonctions réactives du (ou des) bloc(s) PAG et des séquences polyAAO et polyAAI de l'étape 1, peuvent être des fonctions amine ou acide carboxylique. On peut envisager de réaliser la polymérisation conduisant au(x) bloc(s) PAG et/ou au(x) séquence(s) hydrophile(s) PolyAAI et/ou au(x) séquence(s) hydrophobe(s) PolyAAO avant, pendant ou après la formation de la liaison PAG-PolyAAI et/ou PAG-PolyAAO et/ou PolyAAI-PolyAAO.

Toutes ces variantes sont à la portée de l'homme de l'art.

De préférence, dans l'étape 1 :
1.1) on réalise une copolymérisation de monomères formés par des anhydrides de N-CarboxyAminoacides (NCA) d'au moins deux types différents, d'une part, des NCA-pAAI («pBAI » désignant un précurseur d'AAI) et, d'autre part, des NCA-AAO en présence :
   - d'au moins un solvant polaire, de préférence choisi dans le groupe comprenant : la N-MéthylPyrrolidone (NMP), le DiMéthylFormamide (DMF), le DiMéthylsulfOxyde (DMSO), le DiMéthylAcétamide (DMAc), la pyrrolidone ; la NMP étant plus particulièrement préférée ;
   - et éventuellement d'au moins un co-solvant sélectionné parmi les solvants aprotiques (de préférence le dioxanne-1,4) et/ou les solvants protiques (de préférence la pyrrolidone) et/ou l'eau et/ou les alcools, le méthanol étant particulièrement préféré ;
   - on transforme les motifs récurrents pAAI du copolymère précurseur PAA des particules, en motifs récurrents AAI, en mettant en oeuvre une hydrolyse, de préférence acide, pour laquelle on ajoute au milieu organique susdécrit une phase aqueuse acide ;
1.2) on met en oeuvre ou on prépare par polymérisation de monomères alkylène-glycol (de préférence ethylène ou propylène-glycol) au moins un bloc polymère PAG de poly-alkylène-glycol (de préférence de PEG ou PPG); ce bloc PAG étant fonctionnalisé (avantageusement seulement à l'une de ses extrémités) par un groupement réactif, de préférence choisi dans le groupe comprenant les amines (en particulier les amines primaires ou secondaires), les alcools ou les thiols, les acides carboxyliques activées (par réaction préalable avec du dicyclohexylcarbodiimide, carbonyldiimidazole ou autre réactif connu à l'homme du métier) ;
1.3) on ajoute le PAG fonctionnalisé de l'étape 2 au milieu de polymérisation des séquences hydrophiles polyAAI et hydrophobes polyAAO, avant, pendant ou après la polymérisation.

L'étape 1.1 du procédé s'inspire des techniques connues de polymérisation d'anhydrides de N-carboxy-(α-aminoacides (NCA), décrites, par exemple, dans l'article « Biopolymers, 15, 1869 (1976) » et dans l'ouvrage de H.R. KRICHELDORF « α-Aminoacid-N-carboxy-Anhydride and Related Heterocycles » Springer Verlag (1987).

Suivant une variante, lors de l'étape 1.1, on précipite - de préférence dans l'eau- le copolymère poly(AAO)-poly(AAI) obtenu et on recueille ce précipité. Cette variante correspond à un mode discontinu de préparation de particules, dans lequel on isole le copolymère poly(AAO)-poly(AAI) sous forme de précipité formant un produit intermédiaire stable. Ce précipité peut être, par exemple, filtré, lavé et séché.

De manière plus préférée encore, les NCA-pAAI sont des NCA d'acide glutamique ou aspartique O-alkylé, par exemple des NCA-Glu-O-Me, NCA-Glu-O-Et ou NCA-Glu-O-Bz (Me = méthyle - Et = éthyle).

Plus généralement, la préparation des particules peut s'opérer e.g. par addition d'un non-solvant de la fraction hydrophobe à une solution du copolymère amphiphile dissous dans un solvant, avantageusement suite à la synthèse du terpolymère. L'addition de la solution du terpolymère à un non-solvant de la fraction hydrophobe constitue une variante de ce procédé. L'opération consiste préférentiellement à diminuer la solubilité de la fraction hydrophobe pour qu'elle s'agrège, et ce faisant, à former des PV. L'homme de l'art est en mesure de trouver d'autres moyens pour diminuer la solubilité de la fraction hydrophobe du polymère, en modifiant, par exemple, la température, la nature du solvant(s) et du non solvant, ou en combinant différentes techniques.

Par exemple, lors de cette préparation de suspension colloïdale, les copolymères amphiphiles PAG-poly(AAI)-poly(AAO) de l'étape 1 sont placés dans un milieu aqueux dans lequel au moins une partie des PAG est soluble et au moins une partie des AAO est insoluble. Les copolymères PAG-polyAAI-polyAAO existent sous forme de nanoparticules dans ce milieu aqueux.

Une alternative pour préparer la suspension de PV selon l'invention consiste à mettre en présence le solide pulvérulent, tel que décrit ci-dessus en tant que produit et par son procédé d'obtention, avec un milieu aqueux, et en particulier avec de l'eau, non-solvant de la fraction hydrophobe du copolymère amphiphile.
Ainsi, les PV peuvent être obtenues dans l'eau en absence de tout solvant ou de tensioactif.

De préférence, le ou les bloc(s) PAG fonctionnalisé(s) est (sont) introduit(s) avant et/ou au début de la polymérisation, qui se déroule de préférence à une température comprise entre 20 et 120°C à pression atmosphérique normale.

Avantageusement, les PAG de l'étape 1.2 sont des produits commerciaux disponibles (PEG e.g.), ou bien encore sont obtenus de manière connue en soi par polymérisation d'oxyde d'éthylène.

D'autres paramètres, comme la concentration en polymère, la température du mélange réactionnel, le mode d'ajout du polymère hydrophile, l'emploi de pression réduite, la durée de la réaction, etc... sont ajustés selon les effets désirés et bien connus de l'homme de l'art.

La description des caractéristiques des polymères, faite supra dans le cadre de la présentation des particules, peut être intégralement transposée dans le présent exposé relatif au procédé. C'est ainsi que, conformément au procédé selon l'invention, on peut choisir la nature et la quantité des aminoacides récurrents, ainsi que les conditions opératoires, de manière à obtenir différents types de polymères ayant les caractéristiques susvisées.

Pour effectuer l'association (étape 3) d'un ou plusieurs **PA** aux particules, il est possible de mettre en oeuvre plusieurs méthodes conformément à l'invention. Des exemples, non limitatifs, de ces méthodes sont énumérés ci-après.

Selon une première méthode, on effectue l'association de **PA** aux particules par mise en présence d'une phase liquide (aqueuse ou non) contenant le PA avec la suspension colloïdale de particules.

Selon une deuxième méthode, on effectue l'association du **PA** aux particules par mise en présence d'un **PA** à l'état solide avec la suspension colloïdale de particules. Le **PA** solide peut être, par exemple, sous forme de lyophilisat, de précipité, de poudre ou autre.

Selon une troisième méthode, on met en présence le solide pulvérulent (**PAA**), tel que décrit supra en tant que produit et par ses caractéristiques d'obtention, avec une phase liquide (aqueuse ou non) contenant le **PA.**

Selon une quatrième méthode, on met en présence le solide pulvérulent, tel que décrit supra en tant que produit et par ses caractéristiques d'obtention, avec le **PA** sous forme solide. On disperse ensuite ce mélange de solides dans une phase liquide, de préférence une solution aqueuse.

Dans toutes ces méthodes, le **PA** utilisé peut être sous forme pure ou préformulée.

La préparation des **PV** est avantageusement suivie d'unie étape de purification, impliquant des techniques connues de l'homme du métier. Après cette étape facultative de purification, on dispose d'une suspension colloïdale de **PV** que l'on peut utiliser directement, ou qu'il est envisageable d'isoler ou de recueillir par tout moyen physique connu en soi et approprié, comme par exemple : par filtration, par concentration, par ultrafiltration, par séparation par gradient de densité, par centrifugation, par précipitation, éventuellement par ajout de sel, ou bien encore par lyophilisation.

Conformément à l'étape facultative 5, on élimine les impuretés (sels) ainsi que le solvant, par tout traitement de séparation physique approprié, par exemple par diafiltration (dialyse) (étape 4), filtration, modification de pH, chromatographie, distillation. De telles méthodes permettent d'éliminer les sels ou les solvants indésirables.

Pour concentrer (étape 6) ou pour séparer (étape 7) les particules structurées obtenues de leur milieu liquide de suspension, on élimine, éventuellement, la phase aqueuse, par exemple par distillation par séchage (e.g. à l'étuve), par lyophilisation ou tout autre moyen physique convenable : ultrafiltration, centrifugation. On récupère, à l'issue de cette étape 7, un solide pulvérulent, de couleur blanche.

Compte tenu de la taille nanométrique des particules, la suspension peut être filtrée sur des filtres de stérilisation, ce qui permet d'obtenir, aisément et à moindre coût, des liquides médicamenteux injectables stériles. Le fait de pouvoir, grâce à l'invention, contrôler la taille des particules et atteindre des valeurs de diamètre hydrodynamique (Dh) entre 25 et 100 nm, est un atout important.

Selon un autre de ses aspects, l'invention concerne une suspension et/ou un solide pulvérulent, tels que définis ci-dessus et/ou tels qu'obtenus par le procédé présenté supra, cette suspension et ce solide comprenant au moins un principe actif, choisi, de préférence, parmi :
* les vaccins pris à eux seuls ou associés à au moins un antigène;
* les protéines et/ou les peptides, parmi lesquels les plus préférentiellement retenus sont : les hémoglobines, les cytochromes, les albumines, les interférons, les antigènes, les anticorps, l'érythropoiétine, l'insuline, les hormones de croissance, les facteurs VIII et IX, les interleukines ou leurs mélanges, les facteurs stimulants de l'hématopoièse ;

- les polysaccharides, l'héparine étant plus particulièrement sélectionnée ;
- les acides nucléiques et, préférablement, les oligonucléotides d'ARN et/ou d'ADN ;
- des molécules non peptido-protéiques appartenant à diverses classes de chimiothérapie anticancéreuses et, en particulier, les anthracyclines et les taxoïdes ;
- et leurs mélanges.

Enfin, l'invention concerne une spécialité pharmaceutique, nutritionnelle, phytosanitaire ou cosmétique, caractérisée en ce qu'elle comporte une suspension et/ou du solide pulvérulent chargé(s) en **PA** et tels que définis ci-dessus.

Selon un autre de ses objets, l'invention vise, également, l'utilisation de ces **PV** (en suspension ou sous forme solide) chargées en **PA,** pour la fabrication de médicaments du type systèmes à libération contrôlée de **PA.**

Il peut s'agir, par exemple de ceux administrables, de préférence par voie orale, nasale, vaginale, oculaire, sous-cutanée, intraveineuse, intramusculaire, intradermique, intrapéritonéale, ou intracérébrale.

Les applications cosmétiques envisageables sont, par exemple, les compositions comprenant un **PA** associé aux **PV** selon l'invention et applicables par voie transdermique.

Les exemples qui suivent et qui concernent le PA hydrophile formé par l'insuline permettront de mieux comprendre l'invention dans ses différents aspects produit/procédé/application. Ces exemples illustrent la préparation de particules de polyaminoacides chargés ou non en **PA,** de même qu'ils présentent les caractéristiques de structure et les propriétés de ces particules.

### LEGENDES DES FIGURES

Fig 1 - Schéma d'une particule de vectorisation du type de celles adsorbant le **PA.**
Fig 2 - Schéma d'une particule de vectorisation du type de celles encapsulant le **PA.**
Fig 3 - Evolution de la glycémie G (moyenne en % basal) après injection d'une formulation de **PV** (exemple 5) chargée en insuline à raison de 0,6 UI/kg, en fonction du temps t (en heures).
Fig 4 - Photographie de PV (exemple 2) au microscope électronique à transmission.
Fig 5 - Evolution de la glycémie (moyenne en % basal) et de l'insulinémie moyenne I (en mU/l) chez des chiens, en fonction du temps t (en heures), après injection d'une formulation de PV (exemple 6) chargée en insuline à raison de 0,6 UI/kg.
Fig 6 - Evolution de la glycémie G (moyenne en % basal) et de l'insulinémie moyenne I (en mU/l) chez des chiens, en fonction du temps t (en heures), après injection d'une formulation de **PV** (exemple 9) chargée en insuline à raison de 0,6 UI/kg.

### EXEMPLES

### Exemple 1 : Préparation du poly(leucine)12-bloc-poly(glutamate)35-(polyéthylèneglycol)113.

Les techniques utilisées pour la polymérisation des NCA en polymères de structures en blocs ou statistiques sont connues de l'homme de l'art et sont détaillées dans l'ouvrage de H. R. KRICHELDORF « α-aminoacides-N-Carboxy Anhydrides and Related Heterocycles », Springer Verlag (1987). La synthèse suivante précise la synthèse de l'un d'entre eux.

4,96 g de aminoethyl-PEG (masse molaire 5000; Degré de Polymérisation (DP) 113) sont solubilisés dans 120 ml de NMP à 40°C. On y ajoute 1,4ml MeOH puis, 6,4 g de NCA-GluOMe en une fois. Après 1/2h, 1,8 g de NCA-Leu sont ajoutés est la réaction se poursuit pendant 2h. Ensuite, on ajoute au milieu réactionnel de l'acide chlorhydrique dilué et on chauffe à 80°C pendant 24h. A 50°C, le milieu est neutralisé avec de la soude 6N. On dialyse cet intermédiaire contre de l'eau pour éliminer les résidues solubles de faible taille (solvant, sels). La solution purifiée est lyophilisée pour donner une poudre blanche. Rendement 80%.

### Exemple 2 : Préparation du poly(leucine)12-bloc-poly(glutamate)18 -(polyéthylèneglycol)17

2,01 g de aminoethyl-PEG (masse molaire 750; DP 17) sont solubilisés dans 45 ml de NMP à 40°C. On y ajoute 1,5ml MeOH puis, 9 g de NCA-GluOMe en une fois. Après 1/2h, 5 g de NCA-Leu sont ajoutés est la réaction se poursuit pendant 2h. Ensuite, on ajoute au milieu réactionnel de l'acide chlorhydrique dilué et on chauffe à 80°C pendant 24h. A 50°C, le milieu est neutralisé avec de la soude 6N. On dialyse cet intermédiaire contre de l'eau pour éliminer les résidus solubles de faible taille (solvant, sels). La solution purifiée est lyophilisée pour donner une poudre blanche. Rendement 80%.

### Exemple 3: Mise en évidence des nanoparticules par Diffusion de la Lumière (DDL) et par Microscopie Electronique à Transmission (TEM).

10 mg du copolymère obtenu à l'exemple 1 ou 2 sont suspendus dans 10 ml d'eau ou une solution aqueuse de sel. Cette solution est ensuite introduite dans un granulomètre Coulter (ou diffractomètre laser). Les résultats de l'analyse de la granulométrie des différents produits testés sont présentés dans le tableau 1 suivant.

**Tableau 1 - Mesures de la taille des PV**

| **Exemple** | **Polymère** | **Taille (nm)** |
|---|---|---|
| 1 | PEG₁₁₃-(GLU) ₃₅- (LEU)₁₂ | 80 |
| 2 | PBG₁₇- (GLU) ₁₈- (LEU) ₁₂ | 41 |

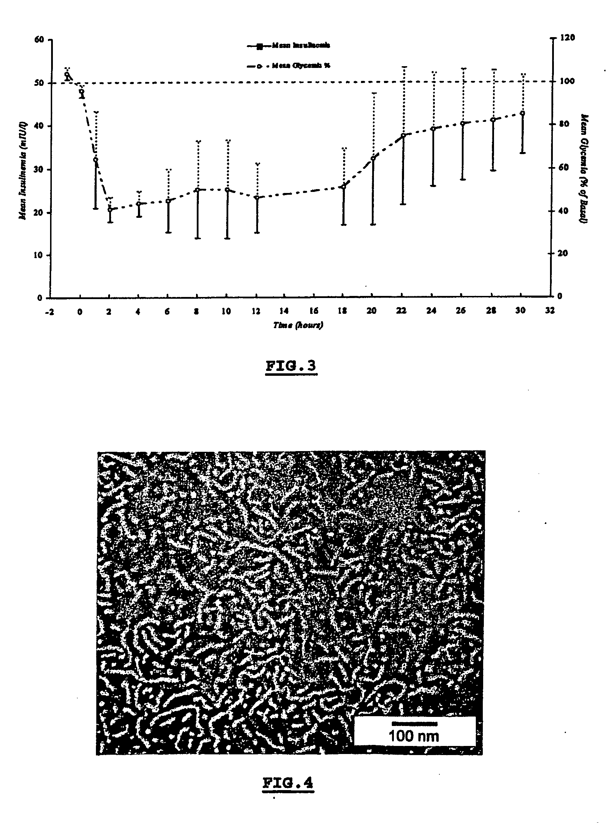

On photographie par ailleurs au microscope électronique à transmission (Fig.4 ci-jointe), les particules de vectorisation PV préparées dans le présent exemple par mise en suspension du copolymère amphiphile de l'exemple 2 dans l'eau.

### Exemple 4 : Test d'association des nanoparticules avec une proteine (l'insuline).

A partir d'une solution tampon phosphate isotonique de pH 7,4, on prépare une solution d'insuline humaine titrée à 1,4 mg/ml correspondant à 40 UI/ml. Dans 1 ml de cette solution d'insuline, on disperse 10 mg du copolymère amphiphile selon l'exemple 1 ou 2. Après 15 heures d'incubation à température ambiante, l'insuline associée aux particules de vectorisation PV et l'insuline libre sont séparées par centrifugation (60 000 g, 1 heure) et ultrafiltration (seuil de filtration 300 000 D). L'insuline libre récupérée dans le filtrat est dosée par Chromatographie Liquide Haute Performance ou par ELISA et l'on en déduit par différence la quantité d'insuline associée. Le tableau 2 suivant rassemble les résultats des mesures de taux d'association effectuées sur différents PV. Le taux d'association exprime le pourcentage d'insuline associée par rapport à l'insuline engagée dans une préparation titrée à 1,4mg/ml d'insuline et 10 mg/ml de PV. Cette valeur est transformée en un taux de chargement qui exprime la quantité maximale en mg d'insuline qui peut s'associer avec 100 mg de PV.

**Tableau 2 - Mesures du taux d'association avec l'insuline pour un mélange 0.14 mg INSULINE/mg PV**

| Exemple | Polymère | Taux max. de chargement mg/100mg **PV** |
|---|---|---|
| 1 | PEG₁₁₃ - (GLU) ₃₅- (LEU)₁₂ | 10 |
| 2 | PEG₁₇- (GLU) ₁₈- (LEU)₁₂ | 19 |

### Exemple 5 : Pharmacocinétique et pharmacodynamie des PV-charges avec l'insuline chez le chien sain à jeun.

La suspension de PV chargée en insuline préparée à l'exemple 4. a été injectée à deux chiens, rendus diabétiques par pancréatectomie totale, et à jeun de la veille au soir. L'administration à 11 heures du matin par voie sous cutanée thoracique de la préparation a été faite à la posologie de 0,5 UI/kg d'insuline par Kg de poids vif de l'animal. Le volume administré est compris entre 0, 18 et 0, 24 ml. Au temps -4, -2, 0, 1, 2, 4, 6, 8, 12, 16, 20, 24, 28, 32, 36, 40, 44 et 48 heures, 1 ml de sang est prélevé par ponction jugulaire sous vide sur tube héparinate de sodium. 30 µl de sang total sont utilisés extemporanément pour mesure de la glycémie. Le tube est ensuite centrifugé, décanté et le plasma stocké à -20° C pour dosage de l'insuline. Les résultats présentés dans la figure 3 ci-après montrent un effet hypoglycémiant important (sur les deux animaux) qui se prolonge au moins jusqu'à 24 heures après l'injection.

**Tableau 3 - Mesures du temps d'action de l'insuline (effet hypoglycémiant) en présence de PV selon l'invention**

| Exemple | Polymère | Temps de retour au niveau basal de la glycémie (en heures) | |
|---|---|---|---|
| | INSULINE SOLUBLE (SANS PV) | 1 | |
| 1 | PEG₁₁₃- (GLU) 35- (LEU)₁₂ | - | - |
| 2 | PEG₁₇-(GLU) ₁₈- (LEU)₁₂ | ≥ 30 | FIGURE 3 |

Cet exemple démontre la non dénaturation de l'insuline en présence de PV selon l'invention.

De plus, cet exemple permet de mettre en évidence l'augmentation à plus de 30h de la durée d'action de l'insuline par rapport à l'insuline non formulée, et donc l'utilité des PV en tant que système retard pour la libération contrôlée de l'insuline. Elle montre également comment il est possible de maîtriser la durée d'action par le choix judicieux du groupement hydrophobe.

### Exemple 6 comparatifs: Préparation du polymère - poly(leucine)40-bloc-(polyéthyléneglycol) 113.

*Synthèse de la poly(Leu)₄₀-PEG:* 10 g de NCA-Leu sont solubilisés dans 150 ml de NMP à 60°C. 5 ml d'une solution de 2 g de aminoéthyl-PEG (Mw 5000) dans 50 ml de NMP sont ajoutés au monomère en une fois. Après 2 h, on vers le milieu réactionnel dans 1L d'eau. Le précipitat formé est filtré, lavé et séché. Rendement >95%.

Le précipitat est dissout dans 100ml d'acide trifluoroacétique, puis on y ajoute 40 ml d'eau sur une période d'une heure. La suspension est ensuite neutralisée à l'aide de la soude, dialysée contre de l'eau pour éliminer les sels ainsi formés, et lyophilisée pour obtenir un produit solide.

### Exemple 7 comparatif : Pharmacocinétique et pharmacodynamie des PV-associée avec l'insuline chez le chien sain à jeun.

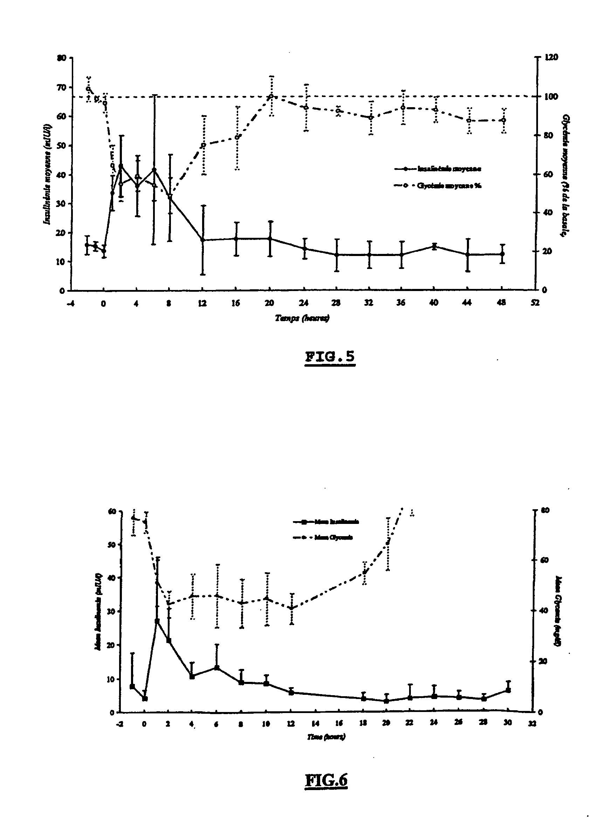

Les **PV** de l'exemple 6 sont formulées puis injectées dans des animaux selon le protocole donné à l'exemple 5. Les résultats présentés dans la figure 5 ci-après montrent un effet hypoglycémiant (sur les deux animaux) et qui se prolonge jusqu'à 20 heures après l'injection.

### Exemple 8 : comparatif de stabilité de la formulation

Les **PV** de l'exemple 6, PEG-Leu₂₅ sont formulées selon l'exemple 7. La formulation, après un repos de 1 mois à +4°C, forme un dépôt de précipitat, qui ne se dissout pas à 35°C, montrant l'instabilité de cette formulation de **PV**.

Les **PV** de l'exemple 1 (poly(leucine)12-bloc-poly(glutamate)35 - poly(éthylèneglycol-)113 sont formulées selon l'exemple 7. La formulation, après un repos de 1 mois à +4°C, reste transparente et ne forme pas de dépôt de précipitat, montrant la stabilité de cette formulation de **PV** et donc l'avantage d'un polymère tribloc PEG-polyAAI-polyAAO par rapport au copolymère dibloc PEG-polyAAO.

### Exemple 9 comparatif: Préparation du polymère - poly(leucine)12-bloc-(glutamate de sodium)35 et son analyse pharmacodynamique.

Le polymère est réalisé selon l'exemple 1 avec la modification suivante : l'aminoéthyl-PEG de Mw = 5000 et de DP = 113 est remplacé par une quantité équivalente en moles d'ammoniaque. Les PV sont isolées, formulées et injectées selon les exemples précédents (exemples 2 et 5) à raison de 50mg de **PV** pour 100UI d'insuline. Les résultats présentés dans la figure 6 ci-après montrent un effet hypoglycémiant (sur les deux animaux) et qui se prolonge jusqu'à 20 heures après l'injection.

### Commentaires:

**tableau 4 comparatif des durées d'action in vivo de l'insuline nue, de l'insuline associée à des systèmes de vectorisation de l'art antérieur (exemples comp 6 et 8) et de l'insuline associée aux particules de vectorisation selon l'invention (exemple 2)**

| Exemple | Polymère | Temps de retour au niveau basal de la glycémie (h) | |
|---|---|---|---|
| | INSULINE SOLUBLE (SANS PV) | 1 | |
| 2 | PEG₁₇- (GLU) ₁₈- (LEU)₁₂ | ≥ 30 | FIGURE 3 |
| 6 comp | PEG₁₁₃-LEU₄₀ | 20 | FIGURE 5 |
| 8 comp | (GLU) ₃₅- (LEu)₁₂ | 20 | FIGURE 6 |

Il ressort de ce tableau que le système selon l'invention (exemple 2) possède une durée d'action in vivo largement supérieure ( + de 30h contre 20h) à celles des systèmes de l'art antérieur (WO 96/29991): exemples 6 et 8.

## Revendications

1. Suspension colloïdale de particules submicroniques susceptibles d'être utilisées, notamment pour la vectorisation de principe(s) actif(s) (PA), ces particules étant des arrangements supramoléculaires individualisés,
**caractérisée en ce que**:
• ces particules sont à base d'au moins un copolymère amphiphile comprenant :
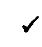 au moins un bloc de polymère(s) hydrophile(s) du type polyéthylène-glycol (PEG) ;
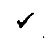 et au moins un copolyaminoacide(s) (PAA) amphiphile linéaire(s), à enchaînements α-peptidiques;
ce copolymère étant constitué de chaînes de copolymères "triblocs linéaires" PEG/ AminoAcide hydrophIle(AAI)/ AminoAcide hydrophObe(AAO),
• et ces particules sont aptes à s'associer en suspension colloïdale à l'état non dissous, avec au moins un PA et à libérer celui-ci, notamment in vivo, de manière prolongée et/ou retardée.

2. Suspension selon la revendication 1, **caractérisée en ce que**:
• l'AminoAcide hydrophile (AAI) est sélectionné(s) dans le groupe comprenant :
➢ les AminoAcides à chaîne(s) ionisable(s), au moins partiellement ionisés, de préférence Glu et/ou Asp et leurs sels et/ou Lys;
➢ et leurs mélanges;
• l'AminoAcide hydrophobe (AAO) est sélectionné(s) dans le groupe comprenant :
➢ les acides aminés neutres naturels, avantageusement ceux du sous-groupe : Leu, Ile, Val, Ala, Pro, Phe, et leurs mélanges ;
➢ des acides aminés neutres, rares ou synthétiques, avantageusement ceux du sous-groupe : norleucine, norvaline et leurs mélanges;
➢ des dérivés des acides aminés polaires avantageusement ceux du sous-groupe : glutamate de méthyle, glutamate d'éthyle, aspartate de benzyle, N-acétyllysine et leurs mélanges;
➢ et leurs mélanges.

3. Suspension selon la revendication 1 ou 2, **caractérisée en ce que** le copolyaminoacide(s) (PAA) amphiphile (s) composant les particules comporte au moins un séquence globalement hydrophile et au moins une séquence globalement hydrophobe.

4. Suspension selon la revendication 3, **caractérisée en ce que** le PAA amphiphile "bloc" comporte avantageusement:
o au moins une séquence globalement hydrophile essentiellement constituée d'aminoacides AAI, et ayant une longueur absolue supérieure ou égale à 5 monomères AAI, de préférence supérieure ou égale à 200 monomères AAI, et plus préférentiellement encore comprise entre 10 et 50 monomères AAI ;
o et au moins une séquence globalement hydrophobe essentiellement constituée d'aminoacides AAO, ayant une longueur absolue supérieure ou égale à 5 monomères AAO, de préférence supérieure ou égale à 10 monomères AAO, et plus préférentiellement comprise entre 10 et 50 monomères AAO.

5. Suspension selon l'une quelconque des revendications 1 à 4, **caractérisée en ce que** le PEG se présente sous la forme d'un bloc dont la longueur absolue est supérieure ou égale à 5 monomères, de préférence comprise entre 5 et 120 monomères, et plus préférentiellement encore comprise entre 5 et 50 monomères.

6. Suspension selon l'une quelconque des revendications 1 à 5, **caractérisée en ce que** les particules sont constituées de chaînes de copolymères "triblocs linéaires" PEG/AAI/AAO, correspondant de préférence à la formule suivante: 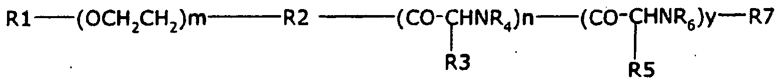 dans laquelle :
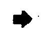 R1 = H, alkyle linéaire ou branché en C₁-C₂₀ (substitué ou non), aryle de préférence benzyle (substitué ou non);
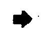 R2 = NH, CO-NH, CS-NH, R8-(CH₂)ₜ-R9 avec R8, R9 choisi indépendamment parmi = OCO, OCONH, NHCO, NHCONH, CONH, COO, NH, CO; t = 1-6, [NHCH(R1)CO-]ₓ;
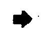 R3 = radicaux identiques ou différents le long de la chaîne et choisis parmi les groupements définissant des aminoacides hydrophiles, ionisables (naturels ou dérivés synthétiques) à savoir de préférence les groupements (CH₂)pC₆H₄OM, (CH₂)ₚCO₂M, (CH₂)ₚN(H_{c}R1_{d})₃X avec p ≥ 1, de préférence = 1 ou 2; a et b sont des valeurs comprise entre 0 et 3 et a+b = 3 ; X étant de préférence un anion chlorure; bromure, sulfate, nitrate, hydrogéno-phosphate, acétate, lactate ;
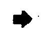 R4 = radicaux identiques ou différents le long de la chaîne et choisis parmi les groupements H, Me ;
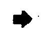 R5 = radicaux identiques ou différents le long de la chaîne et choisis parmi les groupements définissant des aminoacides hydrophobes (naturels ou dérivés synthétiques)à savoir de préférence les à savoir de préférence les groupements H, R1, (CH₂)_{q}C₆H₅, (CH₂)_{q} C₆H₄OR1, (CH₂)_{q} OR1, (CH₂)_{q} CO₂R1, (CH₂)_{q}CON(R1)₂,avec q ≥ 1, de préférence = 1 ou 2;
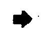 R⁶ = R4 ;
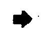 1 R7 = H, RICO avec R1 tel que défini supra, alkyle linéaire ou branché en C₁-C₂₀ (substitué ou non), aryle de préférence benzyle (substitué ou non), hydroxyalkyle en C₁-C₆ H, -(CH₂)_{w}OH, -(CH₂)_{w}CO₂M, X -(CH₂)_{w}(CHR1)_{z}OH, -(CH₂)_{w}NH₂, -(CH₂)_{y}C₆H₄OH, (CH2)yCO-N(R1)₂; R10 = H, Me, (CH₂)ᵥOH, avec w, z et v ≥ 1 et M = métal ou cation, typiquement un métal alcalin comme Na, Li, K, ou NH4, R1NH3 ;
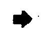 m > 1; n > 3; y > à 0.

7. Suspension selon l'une quelconque des revendications 1 à 6, **caractérisée en ce que** les particules de vectorisation (PV) ont une taille moyenne comprise entre 10 à 500 nm, de préférence entre 10 et 200 nm.

8. Suspension selon l'une quelconque des revendications 1 à 7, **caractérisée en ce que** les particules de vectorisation (PV) comprennent au moins un principe actif.

9. Suspension selon l'une quelconque des revendications 1 à 8, **caractérisée en ce qu'**elle est aqueuse et stable.

10. Solide pulvérulent, **caractérisé en ce qu'**il est obtenu par solidification et mise sous forme de poudre de la suspension selon l'une quelconque des revendications 1 à 9.

11. Procédé de préparation d'un solide pulvérulent obtenu à partir de la suspension selon l'une quelconque des revendications 1 à 9,
**caractérisé en ce que** :
1) on fait réagir au moins un bloc PAG comprenant au moins un monomère alkylène-glycol, avec au moins une séquence hydrophile de PAA comprenant au moins un monomère aminoacide AAI hydrophile et avec au moins une séquence hydrophobe de PAA comprenant au moins un monomère aminoacide AAO hydrophobe, ce bloc de PAG et ces séquences comprenant chacun au moins une fonction réactive de façon à obtenir un copolymère amphiphile "bloc" PAG/polyAAI/polyAAO ;
2) on transfère le copolymère amphiphile bloc PAG/polyAAI/polyAAO obtenu à l'étape 1, dans un milieu non-solvant de la (des) fraction (s) hydrophobe(s) du copolymère amphiphile - de préférence dans l'eau -, ce qui conduit à la formation spontanée de particules de vectorisation de PA ;
3) éventuellement on dialyse le milieu réactionnel pour purifier la suspension aqueuse de particules structurées;
4) éventuellement on concentre cette suspension de l'étape 3;
5) éventuellement on associe au moins un principe actif **PA** avec les particules de l'étape 2, 3 ou 4 ;
6) on élimine le milieu liquide pour recueillir le solide pulvérulent comprenant les particules chargées ou non.

12. Procédé de préparation de la suspension selon l'une quelconque des revendications 1 à 9, **caractérisé en ce que** l'on met en présence d'un milieu aqueux non-solvant de la fraction hydrophobe du copolymère amphiphile, le solide pulvérulent selon la revendication 10 et/ou le solide pulvérulent obtenu par le procédé selon la revendication 11.

13. Procédé de préparation de la suspension selon l'une quelconque des revendications 1 à 9, **caractérisé en ce qu'**il comprend les étapes 1,2,3,4 et éventuellement 5 du procédé selon la revendication 11.

14. Procédé de préparation de la suspension selon la revendication 8, **caractérisé en ce que** l'on effectue l'association du PA aux particules, par mise en présence d'une phase liquide contenant ledit PA hydrophile avec la suspension colloïdale de particules.

15. Procédé de préparation de la suspension selon la revendication 9, **caractérisé en ce que** l'on effectue l'association du PA aux particules par mise en présence dudit PA à l'état solide avec la suspension colloïdale de particules.

16. Procédé de préparation de la suspension selon l'une quelconque des revendications 1 à 9, **caractérisé en ce que** l'on met en présence le solide pulvérulent selon la revendication 10 et/ou le solide pulvérulent obtenu par le procédé selon la revendication 11, avec une phase liquide contenant le PA.

17. Procédé de préparation de la suspension selon l'une quelconque des revendications 1 à 9, **caractérisé en ce que** l'on met en présence le solide pulvérulent selon la revendication 10 et/ou le solide pulvérulent obtenu par le procédé selon la revendication 11, avec le PA sous forme solide et **en ce que** l'on disperse ce mélange de solides dans une phase liquide, de préférence une solution aqueuse.

18. Suspension selon l'une quelconque des revendications 1 à 9 et/ou obtenue par le procédé selon la revendication 11 et/ou solide pulvérulent selon la revendication 10 comprenant un moins un principe actif choisi, de préférence, parmi :
o les vaccins pris à eux seuls ou associés à au moins un antigène
o les protéines et/ou les peptides, parmi lesquels les plus préférentiellement retenus sont : les hémoglobines, les cytochromes, les albumines, les interférons, les antigènes, les anticorps, l'érythropoïétine, l'insuline, les hormones de croissance, les facteurs VIII et IX, les interleukines ou leurs mélanges, les facteurs stimulants de l'hématopoïèse ;
o les polysaccharides, l'héparine étant plus particulièrement sélectionnée ;
o les acides nucléiques et, préférablement, les oligonucléotides d'ARN et/ou d'ADN ;
○ des molécules non peptido-protéiques appartenant à diverses classes de chimiothérapie anti-cancéreuses et, en particulier, les anthracyclines et les taxoïdes ;
o et leurs mélanges.

19. Spécialité pharmaceutique, nutritionnelle, phytosanitaire ou cosmétique, **caractérisée en ce qu'**elle comporte une suspension selon l'une quelconque des revendications 1 à 9 et/ou obtenue par le procédé selon la revendication 11 et/ou du solide pulvérulent selon la revendication 10.

## Claims

1. Colloidal suspension of submicron particles that may be used in particular for the vectorization of active principle(s) (AP), these particles being individualized supramolecular arrangements,
**characterized in that**:
• these particles are based on at least one amphiphilic copolymer comprising:
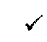 at least one block of hydrophilic polymer(s) of the polyethylene glycol (PEG);
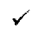 and at least one linear amphiphilic copolyamino acid (PAA), containing α-peptide chains;
this copolymer consisting of "linear triblock" copolymers chains PEG/hydrophllic Amino Acid (PAA)/ hydrophObic Amino Acid (OAA)
• and these particles are capable of associating in colloidal suspension in undissolved form, with at least one AP and of releasing said AP, in particular in vivo, in a sustained and/or delayed manner.

2. Suspension according to claim 1, **characterized in that**:
• the hydrophIlic Amino Acid (IAA) is selected from the group comprising:
➢ Amino Acids with one or more ionizable chain(s), which is (are) at least partially ionized, preferably Glu and/or Asp and salts thereof and/or Lys;
➢ mixtures thereof,
• the hydrophObic Amino Acid (OAA) is selected from the group comprising:
➢ natural neutral amino acids, advantageously those of the sub-group: Leu, Ile, Val, Ala, Pro, Phe, and mixtures thereof;
➢ rare or synthetic neutral amino acids, advantageously those of the sub-group; norleucin, norvalin, and mixtures thereof;
➢ derivatives of polar amino acids, advantageously those of the sub-group: methyl glutamate, ethyl glutamate, benzyl aspartate, N-acetyllysine, and mixtures thereof;
➢ and mixtures thereof.

3. Suspension according to claim 1 or 2, **characterized in that** the amphiphilic copolyamino acid(s) (PAA) of which the particles are composed include(s) at least one sequence that is hydrophilic overall and at least one sequence that is hydrophobic overall.

4. Suspension according to claim 3, **characterized in that** the "block" amphiphilic PAA advantageously includes:
o at least one sequence that is hydrophilic overall, consisting essentially of IAA amino acids, and having an absolute length of greater than or equal to 5 IAA monomers, preferably greater than or equal to 200 IAA monomers and even more preferably between 10 and 50 IAA monomers, and
o at least one sequence that is hydrophobic overall, consisting essentially of OAA amino acids, with an absolute length of greater than or equal to 5 OAA monomers, preferably greater than or equal to 10 OAA monomers and more preferably between 10 and 50 OAA monomers.

5. Suspension according to any one of claims 1 to 4, **characterized in that** the PEG is in the form of a block with an absolute length of greater than or equal to 5 monomers, preferably between 5 and 120 monomers and even more preferably between 5 and 50 monomers.

6. Suspension according to any one of claims 1 to 5, **characterized in that** the particles consist of chains of "linear triblock" copolymers PEG/IAA/OAA, preferably corresponding to the following formula: 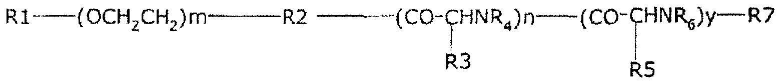 in which:
R1 = H, linear or branched C₁-C₂₀ alkyl (substituted or unsubstituted), aryl, preferably benzyl (substituted or unsubstituted);
→ R2 = NH, CO-NH, CS-NH, R8-(CH₂)ₜ-R9 with R8 and R9 chosen independently from OCO, OCC3NH, NHCO, NHCONH, CONH, COO, NH, CO; t = 1-6, [NHCH(R1)CO-]ₓ;
→ R3 = identical or different radicals along the chain and chosen from the groups defining the ionizable hydrophilic amino acids (natural or synthetic derivatives), i.e. preferably, the groups (CH₂)ₚC₆H₄OM, (CH₂)ₚCO₂M, (CH₂)ₚN(H_{c}R1_{d})₃)X with p ≥ 1, preferably = 1 or 2; a and b are values between 0 and 3 and a+b = 3; X preferably being a chloride, bromide, sulfate, nitrate, hydrogen phosphate, acetate or lactate anion;
→ R4 = identical or different radicals along the chain, chosen from H and Me groups:
→ R5 = identical or different radicals along the chain, chosen from the groups defining the hydrophobic amino acids (natural or synthetic derivatives), i.e. preferably the groups H, R1, (CH₂)_{q}C₆H₅, (CH₂)_{q} C₆H₄OR1, (CH₂)_{q} OR1, (CH₂)_{q} CO₂R1, (CH₂)_{q}CON(R1)₂, with q ≥ 1, preferably = 1 or 2;
→ R6 = R4;
→ R7 = H, R1CO with R1 as defined above, linear or branched C₁-C₂₀ alkyl (substituted or unsubstituted), aryl, preferably benzyl (substituted or unsubstituted), C₁-C₆ hydroxyalkyl, H, -(CH₂)_{w}OH, -(CH₂)_{w}CO₂M, -(CH₂)_{w}(CHR1)_{z}OH, -(CH₂)_{w}NH₂, -(CH₂)_{y}C₆H₄OH, (CH₂)_{y}CO-N(R1)₂; R10 = H, Me, (CH₂)ᵥOH, with w, z and v ≥ 1 and M = metal or cation, typically an alkali metal such as Na, Li or K, or NH₄, R1NH₃;
→ m > 1; n > 3; y > 0.

7. Suspension according to any one of claims 1 to 6, **characterized in that** the vectorization particles (VP) have a mean size of between 10 and 500 nm, preferably between 10 and 200 nm.

8. The suspension according to any one of claims 1 to 7, **characterized in that** the vectorization particles (VP) include at least one active principle.

9. The suspension according to any one of claims 1 to 8, **characterized in that** it is aqueous and stable.

10. Powdered solid, **characterized in that** it is obtained by solidification and conversion into powder form of the suspension according to any one of claims 1 to 9.

11. Process for preparing a powdered solid obtained from the suspension according to any one of claims 1 to 9,
**characterized in that**:
1) at least one PAG block including at least one alkylene glycol monomer is reacted with at least one hydrophilic PAA sequence comprising at least one hydrophilic amino acid IAA monomer and with at least one hydrophobic PAA sequence comprising at least one hydrophobic amino acid OAA monomer, this PAG block and these sequences each including at least one reactive function so as to obtain a PAG/polyIAA/polyOAA "block" amphiphilic copolymer;
2) the PAG/polylAA/polyOAA block amphiphilic copolymer obtained in step 1 is transferred into a medium that is a nonsolvent for the hydrophobic fraction(s) of the amphiphilic copolymer - preferably into water -, which leads to the spontaneous formation of AP vectorization particles;
3) optionally, the reaction medium is dialyzed to purify the aqueous suspension of structured particles;
4) optionally, this suspension from step 3 is concentrated;
5) optionally, at least one active principle AP is combined with the particles from step 2, 3 or 4;
6) the liquid medium is removed to collect the powdered solid comprising the loaded or unloaded particles.

12. Process for preparing the suspension according to any one of claims 1 to 9, **characterized in that** the powdered solid according to claim 10 and/or the powdered solid obtained by the process according to claim 11 is placed in contact with an aqueous medium that is a nonsolvent for the hydrophobic fraction of the amphiphilic copolymer.

13. Process for preparing the suspension according to any one of claims 1 to 9, **characterized in that** it includes steps 1, 2, 3, 4 and optionally 5 of the process according to claim 11.

14. Process for preparing the suspension according to claim 8, **characterized in that** the AP is combined with the particles by placing a liquid phase containing said hydrophilic AP in contact with the colloidal suspension of particles.

15. Process for preparing the suspension according to claim 9, **characterized in that** the AP is combined with the particles by placing said AP in solid form in contact with the colloidal suspension of particles.

16. Process for preparing the suspension according to any one of claims 1 to 9, **characterized in that** the powdered solid according to claim 10 and/or the powdered solid obtained by the process according to claim 11 is placed in contact with a liquid phase containing the AP.

17. Process for preparing the suspension according to any one of claims 1 to 9, **characterized in that** the powdered solid according to claim 10 and/or the powdered solid obtained by the process according to claim 11 is placed in contact with the AP in solid form and **in that** this mixture of solids is dispersed in a liquid phase, preferably an aqueous solution.

18. Suspension according to any one of claims 1 to 9 and/or obtained by the process according to claim 11 and/or the powdered solid according to claim 10, comprising at least one active principle preferably chosen from:
o vaccines, taken alone or combined with at least one antigen;
o proteins and/or peptides, among which the ones most preferably selected are: hemoglobins, cytochromes, albumins, interferons, antigens, antibodies, erythropoietin, insulin, growth hormones, Factors VIII and IX, interleukins or mixtures thereof, and hematopoiesis-stimulating factors;
o polysaccharides, heparin being more particularly selected;
o nucleic acids, and preferably RNA and/or DNA oligonucleotides;
o nonpeptide-protein molecules belonging to various anticancer chemotherapy classes, and in particular anthracyclines and taxoids;
o and mixtures thereof.

19. Pharmaceutical, nutritional, plant-protection or cosmetic specialty product, **characterized in that** it comprises a suspension according to any one of claims 1 to 9 and/or obtained by the process according to claim 11 and/or the powdered solid according to claim 10.

## Patentansprüche

1. Kolloidale Suspension von Submikronpartikeln, die insbesondere zur Vektorisierung eines Wirkstoffs/von Wirkstoffen (W) verwendet werden können, wobei diese Partikel individualisierte supramolekulare Anordnungen sind, **dadurch gekennzeichnet, dass**:
• diese Partikeln auf mindestens einem amphiphilen Copolymer basieren, das Folgendes umfasst:
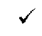 mindestens einen Block aus einem oder
mehreren hydrophilen Polymer(en) des Polyethylenglykol- (PEG-) Typs und
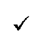 mindestens eine lineare amphiphile Copolyaminosäure (PAA) mit α-peptidischen Bindungen,
wobei dieses Copolymer aus Ketten von "linearen Triblock"-Copolymeren PEG/hydrophIle Aminosäure (AAI)/hydrophObe Aminosäure (AAO) besteht,
• und diese Partikeln in der Lage sind, sich in kolloidaler Suspension im ungelösten Zustand mit mindestens einem W zusammenzulagern und diesen, insbesondere in vivo, auf verlängerte und/oder verzögerte Weise freizusetzen.

2. Suspension nach Anspruch 1, **dadurch gekennzeichnet, dass**:
• die hydrophile Aminosäure (AAI) aus der Gruppe ausgewählt wird, umfassend:
➢ Aminosäuren mit einer oder mehreren ionisierbaren Kette(n), die zumindest teilweise ionisiert ist/sind, vorzugsweise Glu und/oder Asp und deren Salze und/oder Lys
➢ und deren Gemische,
• die hydrophobe Aminosäure (AAO) aus der Gruppe ausgewählt wird, umfassend:
➢ natürliche neutrale Aminosäuren, vorzugsweise diejenigen der Untergruppe: Leu, Ile, Val, Ala, Pro, Phe und deren Gemische,
➢ seltene oder synthetische neutrale Aminosäuren, vorzugsweise diejenigen der Untergruppe: Norleucin, Norvalin und deren Gemische,
➢ Derivate von polaren Aminosäuren, vorzugsweise diejenigen der Untergruppe: Methylglutamat, Ethylglutamat, Benzylaspartat, N-Acetyllysin und deren Gemische;
➢ Une deren Gemische.

3. Suspension nach Anspruch 1 oder 2, **dadurch gekennzeichnet, dass** die amphiphile(n) Copolyaminosäure(n) (PAA), die die Partikeln ausmacht(-en), mindestens eine insgesamt hydrophile Sequenz und mindestens eine insgesamt hydrophobe Sequenz umfasst(-en).

4. Suspension nach Anspruch 3, **dadurch gekennzeichnet, dass** die amphiphile "Block"-PAA vorteilhafterweise Folgendes umfasst:
o mindestens eine insgesamt hydrophile Sequenz, die im Wesentlichen aus AAI-Aminosäuren besteht und eine absolute Länge von mehr als oder gleich 5 AAI-Monomeren, vorzugsweise mehr als oder gleich 200 AAI-Monomeren und noch stärker bevorzugt zwischen 10 und 50 AAI-Monomeren besitzt
o und mindestens eine insgesamt hydrophobe Sequenz, die im Wesentlichen aus AAO-Aminosäuren besteht und eine absolute Länge von mehr als oder gleich 5 AAO-Monomeren, vorzugsweise mehr als oder gleich 10 AAO-Monomeren und stärker bevorzugt zwischen 10 und 50 AAO-Monomeren besitzt.

5. Suspension nach einem der Ansprüche 1 bis 4, **dadurch gekennzeichnet, dass** das PEG in Form eines Blocks vorliegt, dessen absolute Länge mehr als oder gleich 5 Monomere, vorzugsweise zwischen 5 und 120 Monomeren und noch stärker bevorzugt zwischen 5 und 50 Monomeren beträgt.

6. Suspension nach einem der Ansprüche 1 bis 5, **dadurch gekennzeichnet, dass** die Partikeln aus Ketten von "linearen Triblock"-Copolymeren PEG/AAI/AAO bestehen, die vorzugsweise der folgenden Formel entsprechen: 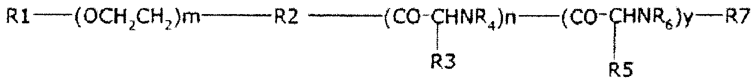 worin:
→ R1 = H, lineares oder verzweigtes (substituiertes oder unsubstituiertes) C₁-C₂₀-Alkyl, (substituiertes oder unsubstituiertes) Aryl, vorzugsweise Benzyl,
→ R₂ = NH, CO-NH, CS-NH, R8-(CH₂)ₜ-R9, wobei R8, R9 unabhängig aus OCO, OCONH, NHCO, NHCONH, CONH, COO, NH, CO ausgewählt werden, t = 1-6, [NHCH(R1)CO-]ₓ,
→ R3 = gleiche oder verschiedene Reste entlang der Kette und ausgewählt aus den Gruppen, die für ionisierbare hydrophile Aminosäuren (natürliche oder synthetische Derivate) charakteristisch sind, d. h. vorzugsweise die Gruppen (CH₂)ₚC₆H₄OM, (CH₂)ₚCO₂M, (CH₂)ₚN(H_{c}R¹_{d}) ₃X, wobei p ≥ 1, vorzugsweise = 1 oder 2, a und b Werte zwischen 0 und 3 sind und a + b = 3, X vorzugsweise ein Chlorid-, Bromid-, Sulfat-, Nitrat-, Hydrogenphosphat-, Acetat- Lactatanion ist,
→ R4 = gleiche oder verschiedene Reste entlang der Kette und ausgewählt aus H, Me,
→ R5 = gleiche oder verschiedene Reste entlang der Kette und ausgewählt aus den Gruppen, die für hydrophobe Aminosäuren (natürliche oder synthetische Derivate) charakteristisch sind, d. h. vorzugsweise die Gruppen H, R1, (CH₂)_{q}C₆H₅, (CH₂)_{q}C₆H₄OR1, (CH₂)_{q}OR1, (CH₂)_{q}CO₂R1, (CH₂)_{q}CON(R1)₂, wobei q ≥ 1, vorzugsweise = 1 oder 2,
→ R6 = R4,
→ R7 = H, R1CO, wobei R1 wie vorstehend definiert ist, lineares oder verzweigtes (substituiertes oder unsubstituiertes) C₁-C₂₀-Alkyl, (substituiertes oder unsubstituiertes) Aryl, vorzugsweise Benzyl, C₁-C₆-Hydroxyalkyl, H, -(CH₂)_{w}OH, -(CH₂)_{w}CO₂M, -(CH₂)_{w}(CHR1) _{z}OH, -(CH₂)_{w}NH₂, -(CH₂)_{y}C₆H₄OH, (CH₂)_{y}CO-N(R1)₂, R10 = H, Me, (CH₂)ᵥOH, wobei w, z und v ≥ 1 und M = Metall oder Kation, gewöhnlich ein Alkalimetall, wie Na, Li, K, oder NH₄, R1NH₃,
→ m > 1, n > 3, y > 0.

7. Suspension nach einem der Ansprüche 1 bis 6, **dadurch gekennzeichnet, dass** die Vektorisierungspartikeln (VP) eine mittlere Größe zwischen 10 und 500 nm, vorzugsweise zwischen 10 und 200 nm aufweisen.

8. Suspension nach einem der Ansprüche 1 bis 7, **dadurch gekennzeichnet, dass** die Vektorisierungspartikeln (VP) mindestens einen Wirkstoff enthalten.

9. Suspension nach einem der Ansprüche 1 bis 8, **dadurch gekennzeichnet, dass** sie wässrig und stabil ist.

10. Pulverförmiger Feststoff, **dadurch gekennzeichnet, dass** er erhalten wird, indem man die Suspension nach einem der Ansprüche 1 bis 9 verfestigt und in Pulverform bringt.

11. Verfahren zur Herstellung eines pulverförmigen Feststoffs ausgehend von der Suspension nach einem der Ansprüche 1 bis 9,
**dadurch gekennzeichnet, dass** man:
1) mindestens einen PAG-Block, der mindestens ein Alkylenglykol-Monomer umfasst, mit mindestens einer hydrophilen PAA-Sequenz, die mindestens ein hydrophiles AAI-Aminosäure-Monomer umfasst, und mit mindestens einer hydrophoben PAA-Sequenz, die mindestens ein hydrophobes AAO-Aminosäure-Monomer umfasst, umsetzt, wobei dieser PAG-Block und diese Sequenzen jeweils mindestens eine reaktive Funktion umfassen, so dass ein amphiphiles PAG/polyAAI/polyAAO-"Block"-Copolymer erhalten wird,
2) das in Schritt 1 erhaltene amphiphile PAG/polyAAI/polyAAO-Block-Copolymer in ein Medium überführt, das kein Lösungsmittel für die hydrophobe(n) Fraktion(en) des amphiphilen Copolymers ist, vorzugsweise in Wasser, was zur spontanen Bildung von W-Vektorisierungspartikeln führt,
3) gegebenenfalls das Reaktionsmedium dialysiert, um die wässrige Suspension der strukturierten Partikeln zu reinigen,
4) gegebenenfalls diese Suspension aus Schritt 3 konzentriert,
5) gegebenenfalls mindestens einen Wirkstoff W mit den Partikeln aus Schritt 2, 3 oder 4 zusammenbringt,
6) das flüssige Medium entfernt, um den pulverförmigen Feststoff, der die beladenen oder nicht beladenen Partikeln umfasst, zu gewinnen.

12. Verfahren zur Herstellung der Suspension nach einem der Ansprüche 1 bis 9, **dadurch gekennzeichnet, dass** man den pulverförmigen Feststoff nach Anspruch 10 und/oder den pulverförmigen Feststoff, der durch das Verfahren nach Anspruch 11 erhalten wird, mit einem wässrigen Medium zusammenbringt, das kein Lösungsmittel für die hydrophobe Fraktion des amphiphilen Copolymers ist.

13. Verfahren zur Herstellung der Suspension nach einem der Ansprüche 1 bis 9, **dadurch gekennzeichnet, dass** es die Schritte 1, 2, 3, 4 und gegebenenfalls 5 des Verfahrens nach Anspruch 11 umfasst.

14. Verfahren zur Herstellung der Suspension nach Anspruch 8, **dadurch gekennzeichnet, dass** man die Zusammenlagerung des W mit den Partikeln durchführt, indem man eine flüssige Phase, die den hydrophilen W enthält, mit der kolloidalen Suspension der Partikeln zusammenbringt.

15. Verfahren zur Herstellung der Suspension nach Anspruch 9, **dadurch gekennzeichnet, dass** man die Zusammenlagerung des W mit den Partikeln durchführt, indem man den W im festen Zustand mit der kolloidalen Suspension der Partikeln zusammenbringt.

16. Verfahren zur Herstellung der Suspension nach einem der Ansprüche 1 bis 9, **dadurch gekennzeichnet, dass** man den pulverförmigen Feststoff nach Anspruch 10 und/oder den pulverförmigen Feststoff, der durch das Verfahren nach Anspruch 11 erhalten wird, mit einer flüssigen Phase, die den W enthält, zusammenbringt.

17. Verfahren zur Herstellung der Suspension nach einem der Ansprüche 1 bis 9, **dadurch gekennzeichnet, dass** man den pulverförmigen Feststoff nach Anspruch 10 und/oder den pulverförmigen Feststoff, der durch das Verfahren nach Anspruch 11 erhalten wird, mit dem W in fester Form zusammenbringt, und dadurch, dass man dieses Gemisch von Feststoffen in einer flüssigen Phase, vorzugsweise einer wässrigen Lösung, dispergiert.

18. Suspension nach einem der Ansprüche 1 bis 9 und/oder erhalten durch das Verfahren nach Anspruch 11 und/oder pulverförmiger Feststoff nach Anspruch 10, umfassend mindestens einen Wirkstoff, der vorzugsweise aus folgenden ausgewählt wird:
o Impfstoffen, allein oder in Kombination mit mindestens einem Antigen;
o Proteinen und/oder Peptiden, unter denen am stärksten bevorzugt die folgenden in Betracht gezogen werden: Hämoglobine, Cytochrome, Albumine, Interferone, Antigene, Antikörper, Erythropoetin, Insulin, Wachstumshormone, die Faktoren VIII und IX, Interleukine oder deren Gemische, Faktoren, die die Hämatopoese stimulieren,
o Polysaccharide, wobei ganz besonders Heparin ausgewählt wird,
o Nukleinsäuren und vorzugsweise RNA- und/oder DNA-Oligonukleotide,
o nicht-peptidisch verbundene Proteinmoleküle, die zu verschiedenen Krebschemotherapie-Klassen gehören und insbesondere Anthracycline und Taxoide,
o und deren Gemische.

19. Pharmazeutische, Ernährungs-, Pflanzenschutz- oder kosmetische Spezialchemikalie, **dadurch gekennzeichnet, dass** sie eine Suspension nach einem der Ansprüche 1 bis 9 und/oder erhalten durch das Verfahren nach Anspruch 11 und/oder des pulverförmigen Feststoffs nach Anspruch 10 enthält.
